# EUROPEAN PATENT APPLICATION

(11) **EP 3 332 721 A1**
(43) Date of publication of application: **13.06.2018**
(21) Application number: 15900302.9
(22) Date of filing: 06.08.2015
(51) Int. Cl.: A61B 17/78

(54) **BONE FIXATION DEVICE FOR USE IN GUIDED GROWTH AND AS A TENSION BAND**

(71) Applicant: Industrias Medicas Sampedro S.A.S., Sabaneta, Antioquia (CO)
(72) Inventor: TORO RESTREPO, Mauricio, Sabaneta Antioquia (CO)
(74) Representative: Martin Santos, Victoria Sofia
(86) International application number: PCT/IB2015/055982
(87) International publication number: WO 2017/021762

(57) **Abstract**

The present invention relates to a design concept for a bone fixating device that can be used both for closing the physys and also for fixing fractures by means of a tension band, wherein said device is based on the concept of two fixed ends joined by flexible wire, which facilitates both its installations and use, which also allows to perform a minimally invasive surgery due to the reduced size of the implant, whose profile must be as low as possible in order to avoid tissue damage during surgery. Thus, the design of the device of the present invention allows the fastening screws to be located as far as possible from the physys, i.e. in the safe zone, reducing ischemia in the physys and, also, allowing for the surgery to be handled percutaneously.

## Description

### TECHNICAL FIELD

The present invention relates to a design concept for a bone fixating which can be applied to two surgical indications: the first one is for closing the epiphyseal plate on the long bones, thus obtaining a guided growth and correcting angular deformities and growth-related bone dissymmetry, and the second is a tension band, used for fixing avulsion fractures whose biomechanical principle is to transform traction forces into compressive forces to achieve fragment fixation and fracture reduction.

Therefore, for application to closing an epiphyseal plate the design of the present invention lets the fastening screws to be located as far away from the epiphyseal plate as possible, i.e. in the safe zone, thus reducing ischemia of the epiphyseal plate and, also, allows for a surgical procedure that can be handled percutaneously, which is ideal for the patient since recovery is much easier and it reduces the risks associated with invasive surgery.

To apply the design as a stress band, the present invention allows for implantation of a single device, preventing the intra-surgical manipulation of several wires and cables thus reducing the surgical time.

### BACKGROUND OF THE INVENTION

Currently, the plates for bone growth control are commonly used with the goal of aiding in the correction of angular deformities or bone dissymmetry when an extremity or bone grows in a larger proportion than the other.

In this sense, most of the plates currently used are based on static systems that allow for variation of the angle by the use of spherical-head screws and/or hinges that move as the bone grows, hence controlling its growth. Nonetheless, these systems are not the most desirable because they are based on devices or parts that are too rigid and can cause lesions on the tissues and/or do not obtain the desired result, since it depends directly on the function of the hinges or the spherical-head screws as such, but do not count with any type of device or system that generates a flexible resistance to the bone and lets a more precise control of bone growth.

In view of the above, in the state of the art there is a plurality of disclosures related to bone fixating plates, among which there is the document US 2010004652 that discloses an orthopedic device for correcting an angular deformation of a bone structure having a growth plate, where the device includes hinge members connected by a hinge joint and the device adapts to be mounted on the bone structure with the pivot joint located on the growth plate. Thus, the pivot joint with the growth plate promotes an asymmetric growth of the growth plate to correct this angular deformity.

Nonetheless, the plate described in this anteriority is based on the concept of a hinge system for joining the ends, i.e. with a pivot point, which is undesirable because it requires a series of adjustments and later procedures that are uncomfortable and undesirable for the patient.

On the other hand, there is the document US 2010004691 which shows a growth control device that includes a bone plate having a defined profile with steps for a first level, a second level and an intermediate ramp connecting the first level with the second level. In this way, the first level includes a first threaded orifice to receive a first bone fastener and the second level includes a second threaded orifice to receive a second bone fastener, wherein the bone pate includes a proximal pair of female side notches, a distal pair of female side notches, a proximal guidance orifice and a distal guidance orifice.

Nonetheless, the device described in this document presents the disadvantage that it does not disclose any type of coupling between the plates, but instead it recites a single continuous pate that can be manufactured with a flexible material, which requires expensive manufacturing costs that are not suitable for all patients since they require a specific material and in most cases the surgery can be invasive.

Regarding the devices used as tension bands, in the state of the art we find document US 2010/268278, with relates to a vertebral stress band assembly that can join vertebral bodies to connect one vertebra to another, hold the band in an approximately preferred position by applying tension to the band during insertion or limit, impede, inhibit, reduce or interfere with separation of one vertebra from another and, further, block, impede, interfere, inhibit, reduce o present an obstacle to dislocation of a spine implant between the vertebrae to which it is joined.

Finally, we have document EP 2724681 which discloses an implant for bone alignment, which includes a first bone fastener with a first bone coupling adapted to be fixed to the metaphysiary bone and a second bone fastener with a second bone coupling adapted to by fixed on a dyphysiary bone. Thus, a bond connecting both fasteners extends along the physys. Alternatively, the implant in this document adapts to be fixed on both diphysiary sections of two vertebral bodies, and acts as a flexible body between the two previously mentioned bone sections during bone growth. In this manner, the implants are designed to adapt and deform during the process of bone realignment.

According to the above, it is evident for a person expert in the subject matter there is a need in the state of the art for designing and implementing a bone fixating device with a flexible system that can be used both for growth control, as well as for fixating bone fragments by means of a tension band where said system is minimally invasive and the surgery can be carried out in an external or percutanneally and thus create the least possible amount of inconvenience and discomfort as possible to the patient, where at the same time it allows for the use of cortical or locking screws.

### BRIEF DESCRIPTION OF THE DRAWINGS

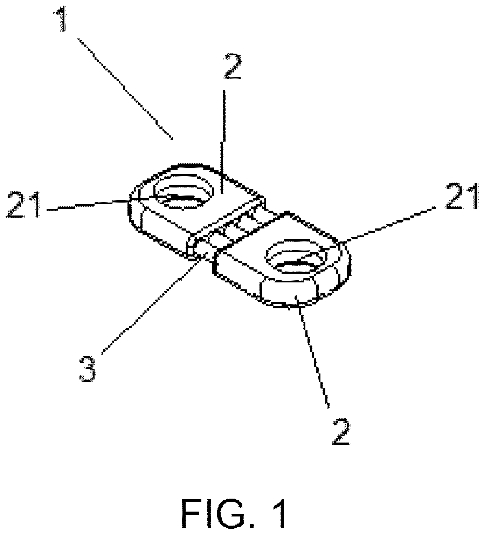

Figure 1 corresponds to a perspective view of the growth plate of the present invention.

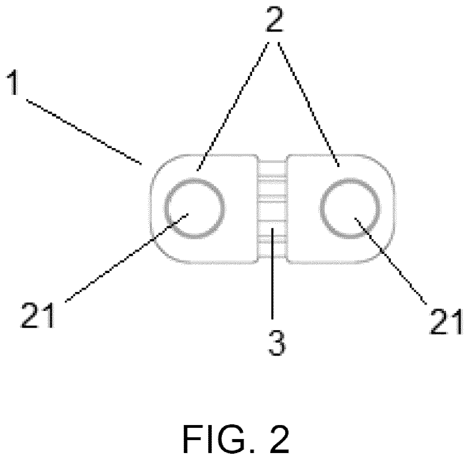

Figure 2 corresponds to a top plane view of the bone fixating device of Figure 1.

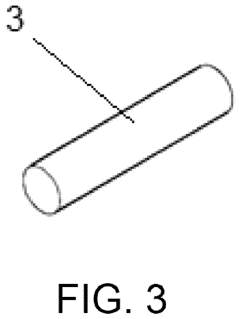

Figure 3 corresponds to a detailed view of the coupling cables of the plate of Figure 1.

### DETAILED DESCRIPTION OF THE INVENTION

The bone fixation device (1) of the present invention is characterized in that it comprises:
- A couple of ends (2) joined directly to the patient's bone, wherein said ends comprise at least one orifice (21) for inserting the screws that are going to fasten the device (1) to the bone; and
- A flexible coupling portion (3) that joins the ends (2), which is comprised by a plurality of cables or wires that deform according to the treatment needs.

Thus, the ends (2) of the plate (1) correspond to the rigid sections and have at least one orifice (21) by which the screws that will be directly fastened to the bone of the patient will be passed, which allows the screws to be located as far as possible from the physys, i.e. in the safe zone.

In a preferred embodiment, these two ends (2) are joined together and form the device (1) through a join portion (3) which is flexible and is made of a plurality of cables or wires, which allow the device to be angularly deformed maintaining its axial resistance and guiding thereby the growth of the bone in a patient.

In another preferred embodiment, the join portion (3) comprises cables or wires whose amount and length directly depends on the needs of the type of procedure to be performed.

In another more preferred embodiment, both the ends (2) of the plate and the cables or wires are preferably manufactured in biocompatible stainless steel.

Finally, the device (1) and specifically its ends (2), have a low profile and the screws being inserted therethrough are conventional screws as those used in any other type of orthopedic procedure, which allows that when the insertion of the device (1) is made, a minimally invasive surgery is carried out and it can be handled in a percutaneous manner.

## Claims

1. A bone fixation device (1), **characterized by** comprising:
• a couple of ends (2) joined directly to the patient's bone, wherein said ends comprise at least one orifice (21) for inserting the screws fastening the device (1) to the bone; and
• a flexible coupling portion (3) that joins the ends (2), which is comprised by a plurality of cables or wires that contract or stretch.

2. The device (1) according to claim 1, **characterized in that** the ends (2) are rigid sections.

3. The device (1) according to claim 1 or 2, **characterized in that** the cables or wires forming the flexible portion (3) are braided with a variable resistance.

4. The device (1) according to any of the preceding claims, **characterized in that** it is manufactured in biocompatible stainless steel.

5. The device (1) according to any of the preceding claims, **characterized in that** specifically its ends (2) have a low profile and the screws being inserted therethrough are cortical screws.
